## Europäisches Patentamt
## European Patent Office
## Office européen des brevets

(19)

(11) Veröffentlichungsnummer: **0 013 306**
**B1**

(12) **EUROPÄISCHE PATENTSCHRIFT**

(45) Veröffentlichungstag der Patentschrift:
03.03.82

(51) Int. Cl.³: **G 01 N 33/68**, G 01 N 33/54

(21) Anmeldenummer: **79104289.8**

(22) Anmeldetag: **02.11.79**

(54) Verfahren zur Bestimmung eines opsonierenden oberflächenbindenden alpha-2-Glykoproteins.

(30) Priorität: **15.01.79 DE 2901327**

(43) Veröffentlichungstag der Anmeldung:
**23.07.80 Patentblatt 80/15**

(45) Bekanntmachung des Hinweises auf die Patenterteilung:
**03.03.82 Patentblatt 82/9**

(84) Benannte Vertragsstaaten:
**AT BE CH DE FR GB IT LU NL SE**

(56) Entgegenhaltungen:
**DE-A-2 692 068**

(73) Patentinhaber: **BOEHRINGER MANNHEIM GMBH,
Sandhofer Strasse 112-132 Postfach 31 01 20,
D-6800 Mannheim 31-Waldhof (DE)**

(72) Erfinder: **Albert, Winfried, Dr., Moosstrase 10, D-8121 Pähl
(DE)**

(74) Vertreter: **Weickmann, Heinrich, Dipl.-Ing Patentanwälte
Dipl.Ing.H.Weickmann et al, Dipl.Phys.Dr.K.Fincke
Dipl.Ing.F.A.Weickmann Dipl.Chem.B.Huber,
Dr.-Ing.H.Liska Möhlstrasse 22, D-8000 München 86 (DE)**

ACTORUM AG.

EP 0 013 306 B1

## Verfahren zur Bestimmung eines opsonierenden oberflächenbindenden alpha-2-Glykoproteins

Die Erfindung betrifft ein Verfahren zur Bestimmung eines opsonierenden, oberflächenbindenden $\alpha_2$-Glykoproteins, welches im folgenden als $\alpha_2$-SB-Glykoprotein bezeichnet wird, und zwar insbesondere zur Bestimmung in Körperflüssigkeiten.

$\alpha_2$-SB-Glykoprotein ist eine Substanz, welche man im Blut und Organstroma findet. Das Serumprotein hat die elektrophoretische Beweglichkeit eines $\alpha_2$-Globulins und einen Sedimentationskoeffizienten von 12 bis 14 S. Sein Molekulargewicht beträgt etwa 400 000 bis 440 000. Es besteht aus zwei Untereinheiten, welche durch Disulfidbrükken verbunden sind. Die Konzentration im Plasma von Gesunden liegt bei 300 bis 500 µg/ml.

Das $\alpha_2$-SB-Glykoprotein zeichnet sich vor allem durch besondere Adhäsionseigenschaften aus und vermag wie ein Klebstoff andere Substanzen, z.B. Zelldebris zu binden.

$\alpha_2$-SB-Glykoprotein ist ein Substrat des aktivierten Gerinnungsfaktors XIII, d.h. des Faktors XIII a) (Plasma-Transglutaminase, Fibrinoligase). Es bindet Fibrinogen und Fibrin, vor allem in der Kälte. Da es während der Gerinnung mit Fibrin reagieren kann, findet man im Serum üblicherweise geringere Konzentrationen als im Plasma.

Eine physiologische Eigenschaft des $\alpha_2$-SB-Glykoproteins ist seine opsonierende Wirkung. Zum Beispiel wird die Aufnahme von partikulärem Material durch das reticuloendotheliale System (RES) gefördert. Daher wird eine direkte Korrelation zwischen $\alpha_2$-SB-Glykoprotein-Spiegel im Plasma und der Aktivität des RES angenommen.

Eine Abnahme des $\alpha_2$-SB-Glykoprotein-Spiegels tritt bei bestimmten Erkrankungen auf. Erhöhte Werte wurden bei Erkrankungen des Bindegewebssystems und bei metastasierenden Tumoren in fortgeschrittenen Stadien beobachtet.

Man nimmt an, dass die Entfernung beschädigten autologen Gewebes und von zirkulierenden kolloidalen Bestandteilen, wie z.B. löslichem Fibrin durch nicht immunologische Opsonierung ein wichtiger physiologischer Vorgang ist. Aus diesem Grunde sollte eine beträchtliche Abnahme von $\alpha_2$-SB-Glykoprotein zu Organversagen bei schwer erkrankten Patienten führen. Die Bestimmung des $\alpha_2$-SB-Glykoprotein-Spiegels ist daher von diagnostischem Interesse.

Verfahren zur qualitativen und quantitativen Bestimmung dieses Proteins sind bekannt. Saba et al (J. Reticuloendothel. Soc. 3, 398 (1966) ) beschreiben einen radiologischen Phagozytose-Test zur semiquantitativen Bestimmung eines opsonierenden Serumfaktors, welcher die Phagozytose von partikulärem Material in die Kupffer'schen Zellen der Leber stimuliert. In diesem Testsystem wurde die Aufnahme von radioaktiv markierten, gelatineüberzogenen Lipidmizellen in Leberschnitten gemessen. Ein immunologisches Nachweisverfahren beschreiben Blumenstock, F.A. et al in J. Reticuloendothel. Soc. 23, 119 (1978). Hierbei wird das $\alpha_2$-SB-Glykoprotein mittels eines Elektro-Immunoassays (Rocket-Elektrophorese) bestimmt. In Hoppe-Seylers, Z. Physiol. Chemie 359, 247 (1978) wird eine quantitative Bestimmungsmethode für CIG (entspricht dem $\alpha_2$-SB-Glykoprotein) angegeben, in der die Affinität von CIG zu 125-J-markiertem Kollagen ausgenutzt und der gebildete Komplex mit Anti-CIG-Antiserum ausgefällt wird. In Int. J. Cancer 20, 1 (1977) wird ein Enzym-Immunoassay beschrieben, wobei Plastikgläschen mit Gelatine oder spezifischem Antikörper gegen Fibronectin überzogen werden und nach der Bindung von Fibronectin (entspricht $\alpha_2$-SB-Glykoprotein) an die Gläschenwand eine Inkubation mit enzymmarkiertem Anti-Fibronectin-Antikörper angeschlossen wird (Sandwich-Prinzip). Aus J. Biol. Chem. 245, 5728 (1970) ist ferner eine immunelektrophoretische Methode zur quantitativen Bestimmung bekannt.

Die bekannten Methoden haben alle den Nachteil, dass sie einerseits einen relativ grossen Arbeitsaufwand erfordern und andererseits die Durchführung der Tests einen Zeitraum von ca. 20 bis 24 Stunden in Anspruch nimmt. Darüber hinaus sind mehrere Arbeitsschritte erforderlich.

Obwohl daher $\alpha_2$-SB-Glykoprotein schon lange quantitativ bestimmt werden kann (1970), ist es bisher noch nicht gelungen, für die klinische Praxis einen einfacheren und rascher durchzuführenden Test zu entwickeln. Wegen der besonderen physiologischen Eigenheiten und den sich daraus ergebenden diagnostischen Konsequenzen wäre aber eine rasche Testdurchführung sehr wünschenswert. Ein schnelles Ergebnis ist besonders bei akuten Fällen zum Zwecke der klinischen Verlaufskontrolle und kontinuierlichen Überwachung erforderlich.

Der Erfindung liegt daher die Aufgabe zugrunde, ein Verfahren zur Bestimmung des $\alpha_2$-SB-Glykoproteins zu entwickeln, welches sich innerhalb weniger Minuten und auf möglichst einfache Weise durchführen lässt und damit erst die Voraussetzung schafft, die kurzfristigen Mengenveränderungen dieser Substanz zu erfassen und für die Diagnose und Behandlung auswertbar zu machen.

Die Lösung dieser Aufgabe gelingt erfindungsgemäss durch ein Verfahren zur Bestimmung eines opsonierenden, oberflächenbindenden $\alpha_2$-Glykoproteins, welches dadurch gekennzeichnet ist, dass man eine gepufferte Lösung von Antikörper gegen das opsonierende, oberflächenbindende $\alpha_2$-Glykoprotein mit der zu untersuchenden Probe versetzt und die sich bildende Trübung in bestimmten Zeitintervallen turbidimetrisch oder nephelometrisch bestimmt.

Überraschenderweise hat sich gezeigt, dass es möglich ist, das $\alpha_2$-SB-Glykoprotein nach einer immunologischen Methode turbidimetrisch zu bestimmen, obwohl man angesichts der klebenden Eigenschaften dieser Substanz erwarten musste, dass Fremdsubstanzen mitgerissen werden und damit die Proportionalität zwischen der Menge des zu bestimmenden Proteins und der auftretenden Trübung nicht mehr gewährleistet ist und darüber hinaus auch Flockungs- oder Zusammenballungseffekte auftreten, die eine quantitative Bestimmung durch Trübungsmessung unmöglich machen würden. Dies wird auch durch die Be- zeichnung «opsonierend» zum Ausdruck ge- bracht, die in direkter Übersetzung «zum Essen zubereiten» bedeutet. Dies soll zum Ausdruck bringen, dass dieses Protein sozusagen zur Erkennung zu entfernender Substanzen dient und durch seine Anhaftung die Zellen des reticuloendothelialen Systems dazu bringt, diese Substanzen mit dem daran haftenden opsonierenden Protein «aufzufressen».

Das erfindungsgemässe Verfahren wird zweckmässig bei pH-Werten zwischen 5 und 9,5, vorzugsweise zwischen 6,5 und 8,5 durchgeführt. Geeignet sind die in diesem Bereich puffernden Substanzen. Bevorzugt werden Puffer mit geringer Ionenstärke, bis etwa 100 mmolar. Jedoch können auch Pufferlösungen grösserer Ionenstärke verwendet werden.

In einer bevorzugten Ausführungsform der Er- findung wird Polyäthylenglykol zugesetzt. Geeig- net sind die wasserlöslichen Polyäthylenglykol- sorten. Ein besonders geeigneter Mengenbereich sind 1 bis 5 Gew.-% Polyäthylenglykol, bezogen auf die Testlösung.

Das Verfahren wird zweckmässig bei Temperaturen zwischen 15 und 35 °C, vorzugsweise zwischen 20 und 30 °, durchgeführt. Bei niedrigeren Temperaturen verläuft die Fällung wesentlich langsamer, bei höheren Temperaturen tritt bereits wieder eine Dissoziation des Immunkomplexes auf.

Die Messung selbst erfolgt bei beliebigen Wellenlängen, wie sie für turbidimetrische Bestimmungen bekannt sind. Kurzwellige, wie z.B. 334, 360 und 405 nm, werden bevorzugt. Auch die ne- phelometrische Bestimmung lässt sich an- wenden.

Die Messung erfolgt in bestimmtem Zeitintervall, beispielsweise nach 1 Minute und nach 10 Minuten. Die Extinktionsdifferenz der gemessenen Werte ist dem Gehalt an $\alpha_2$-SB-Gylkoprotein direkt proportional.

Die Herstellung des Antikörpers bzw. Antiserums erfolgt in der üblichen Weise durch Injektion des als Antigen dienenden $\alpha_2$-SB-Glykoproteins intradermal bzw. intramuskulär in wiederholten Gaben an geeignete Versuchstiere, beispielsweise Kaninchen, Schafe oder Ziegen. Die Verabreichung kann mit üblichen immunologischen Adjuvantien, z.B. Freund' Adjuvans, erfolgen.

Aus Humanplasma wurde $\alpha_2$-SB-Glykoprotein durch Affinitätschromatographie entfernt und das Plasma dann mit Glutardialdehyd zu einem Gel vernetzt. Das Gel wurde homogenisiert und mit dem Homogenat die aus den Versuchstieren gewonnenen Antiseren bis zur immunologischen Monospezifität (überprüft durch Immundiffusion und Immunelektrophorese) absorbiert. Die auf diese Weise nicht absorbierten verbleibenden Antikörper gegen das $\alpha_2$-SB-Glykoprotein wurden im Verfahren eingesetzt.

Das so hergestellte Antiserum wird mit dem oben beschriebenen Puffer auf die gewünschte Konzentration verdünnt, gegebenenfalls mit Po- lyäthylenglykol und Stabilisierungsmitteln ver- setzt und in flüssiger oder lyophilisierter Form aufbewahrt. Geeignete Stabilisierungsmittel sind beispielsweise Mannit, Alkaliazid, Serumalbumin und dergleichen.

Das erfindungsgemässe Verfahren eignet sich sowohl zur Durchführung im manuellen Test als auch für Analysenautomaten. Dies wird durch die beigefügte Zeichnung näher erläutert. In dieser stellen dar:

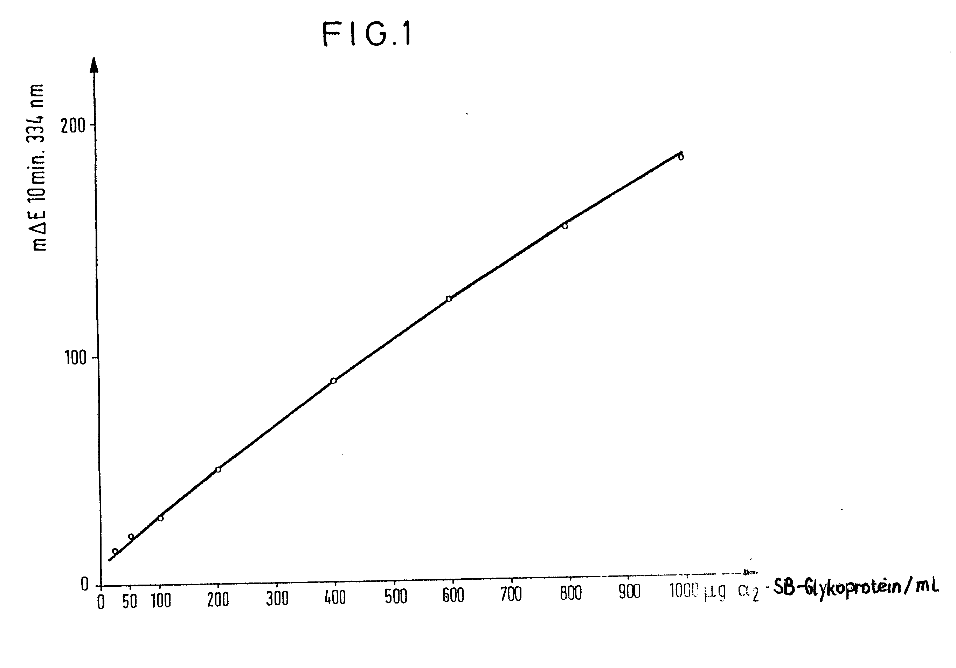

Fig. 1 eine Standardkurve, welche mit dem manuellen Test erstellt wurde,

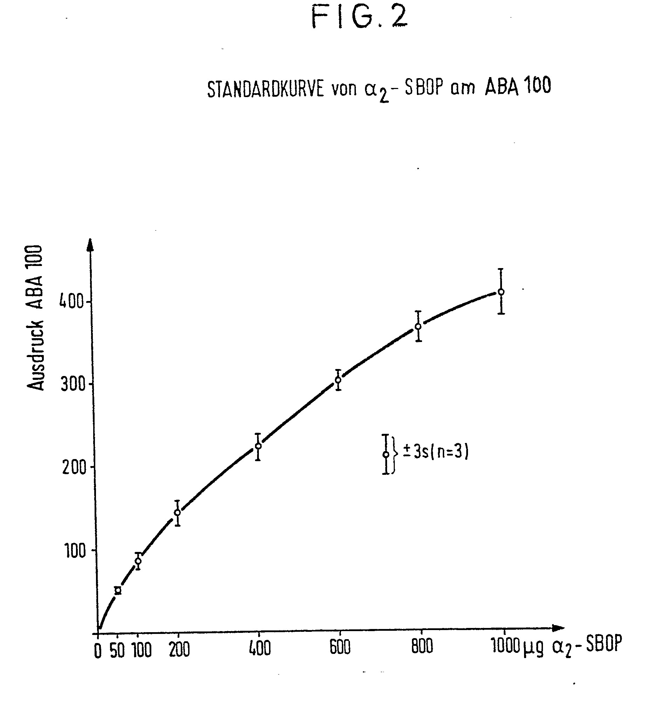

Fig. 2 eine Standardkurve, welche auf einem Analysenautomaten (ABA-100) erstellt wurde,

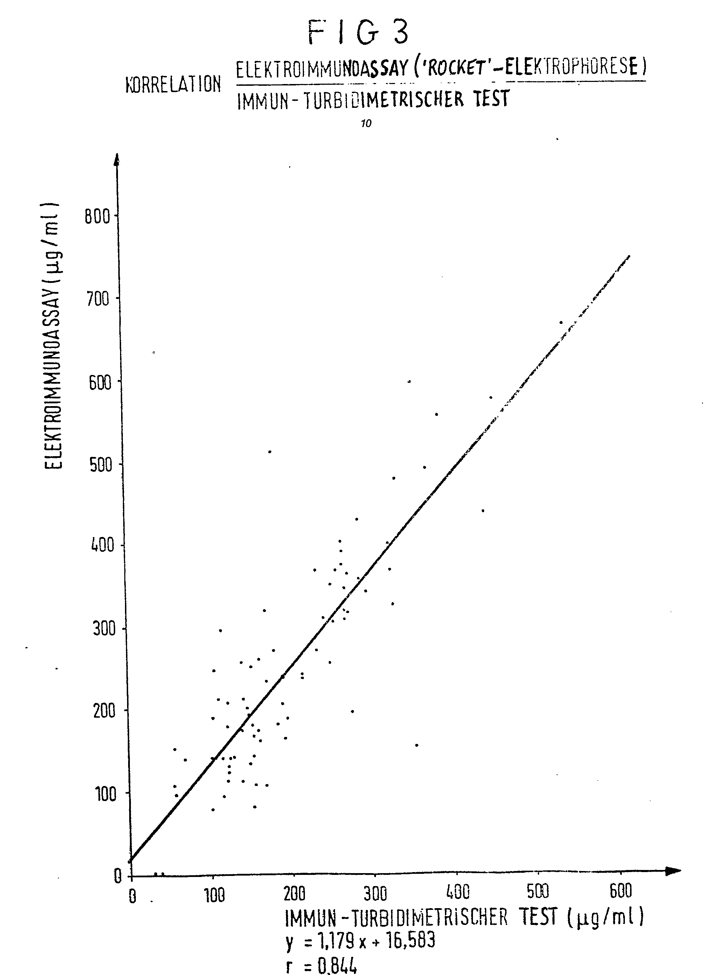

Fig. 3 zeigt die Korrelation zwischen der erfin- dungsgemässen Bestimmung von $\alpha_2$-SB-Glyko- protein in Humanplasma in manueller Durchfüh- rung mit einer immunelektrophoretischen Metho- de (Rocket-Elektrophorese).

Die Bestimmung erfolgt durch Vergleich mit einer Standardlösung von bekanntem $\alpha_2$-SB-Gly- koprotein.

Das erfindungsgemässe Verfahren ermöglicht eine rasche und genaue Bestimmung des $\alpha_2$-SB- Glykoproteins, welche nur wenige Minuten benötigt und sehr einfach mit üblichen photometrischen Geräten durchgeführt werden kann. In gleicher Weise eignet sie sich auch für Analysenautomaten.

Das folgende Beispiel erläutert die Erfindung weiter.

Beispiel

A. Herstellung des Antiserums

$\alpha_2$-SB-Glykoprotein wurde nach der in Biochem. Biophys. Acta 534, 210 (1978) beschriebenen Methode durch Affinitätschromatographie an Gelatine, gebunden an vernetzte Agarose, isoliert.

Kaninchen, Schafe und Ziegen wurden nach folgendem Schema immunisiert:

| Tag | Menge | Applikationsart | Adjuvans |
|-----|-------|-----------------|----------|
| 1 | 500 µg | intradermal an mehreren Stellen | Freund |
| 7 | 100 µg | intramuskulär | Freund |
| 21 | 100 µg | intramuskulär | Freund |
| 49 | 100 µg | intramuskulär | Freund |
| + | 100 µg | intramuskulär | Freund |

+) in monatlichen Abständen

Zur Reinigung der aus den Versuchstieren erhaltenen Antiseren wurde wie folgt vorgegangen: 100 ml Humanplasma wurden durch Affinitäts- chromatographie von $\alpha_2$-SB-Glykoprotein befreit und dann nach dem in Immunochemistry 6, 53 (1969) beschriebenen Verfahren mit Glutardialdehyd zu einem Gel vernetzt, welches homogenisiert wird. Aus den Versuchstieren gewonnenes Antiserum wird über das Gel filtriert. Nach Abtrennung des Gels wies das verbliebene Antiserum immunologische Monospezifität auf.

B. Testdurchführung

Eine Standardlösung von $\alpha_2$-SB-Glykoprotein wurde hergestellt durch Auflösen bestimmter Mengen in 0,15 Mol/l phosphatgepufferter Kochsalzlösung mit 1% Rinderserumalbumin und 0,1% Natriumazid.

Das Antiserum wurde vor Gebrauch mit 0,66 mMol/l Phosphatpuffer, pH 7,4, welcher 2,5% Polyäthylenglykol 6000 und 0,1% $NaN_3$ enthielt, auf die gewünschte Verdünnung gebracht.

Zur Testdurchführung wurden 500 µl des verdünnten Antikörpers in eine Küvette von 1 cm Schichtdicke gebracht und mit 10 µl Probelösung oder Standard versetzt und dann bei 20 bis 25 °C die Extinktion gegen Luft bei Hg 334 nm in einem handelsüblichen Photometer 1 Minute und 10 Minuten nach dem Vermischen gemessen. Der Gehalt an $\alpha_2$-SB-Glykoprotein ergibt sich durch Vergleich der Extinktionsdifferenz mit einer in gleicher Weise erhaltenen Standardkurve.

**Patentansprüche**

1. Verfahren zur Bestimmung eines opsonierenden, oberflächenbindenden $\alpha_2$-Glykoproteins, dadurch gekennzeichnet, dass man eine gepufferte Lösung von Antikörper gegen das opsonierende oberflächenbindende $\alpha_2$-Glykoprotein mit der zu untersuchenden Probe versetzt und die sich bildende Trübung in bestimmten Zeitintervallen turbidimetrisch oder nephelometrisch bestimmt.

2. Verfahren nach Anspruch 1, dadurch gekennzeichnet, dass eine Pufferlösung von pH 5 bis 9,5 verwendet wird.

3. Verfahren nach Anspruch 2, dadurch gekennzeichnet, dass eine Pufferlösung pH 6,5 bis 8,5 verwendet wird.

4. Verfahren nach einem der Ansprüche 1 bis 3, dadurch gekennzeichnet, dass ein wasserlösliches Polyäthylenglykol zugesetzt wird.

5. Verfahren nach Anspruch 4, dadurch gekennzeichnet, dass 1 bis 5 Gew.-% Polyäthylenglykol zugesetzt werden.

6. Verfahren nach einem der vorhergehenden Ansprüche, dadurch gekennzeichnet, dass die Bestimmung bei einer bestimmten Temperatur zwischen 15 und 35 °C durchgeführt wird.

**Revendications**

1. Procédé pour la détermination d'une $\alpha_2$-glycoprotéine opsonique apte à se lier à une surface, caractérisé en ce que l'on ajoute à une solution tamponnée d'anti-corps envers l'$\alpha_2$-glycoprotéine opsonique apte à se lier à une surface l'échantillon à étudier et que l'on détermine le trouble qui se forme à des intervalles de temps déterminés par turbidimétrie ou néphélométrie.

2. Procédé selon la revendication 1, caractérisé en ce que l'on utilise une solution tampon de pH 5 à 9,5.

3. Procédé selon la revendication 2, caractérisé en ce que l'on utilise une solution tampon de pH 6,5 à 8,5.

4. Procédé selon l'une des revendications 1 à 3, caractérisé en ce que l'on ajoute un polyéthylèneglycol soluble dans l'eau.

5. Procédé selon la revendication 4, caractérisé en ce que l'on ajoute 1 à 5% en poids de polyéthylèneglycol.

6. Procédé selon l'une des revendications précédentes, caractérisé en ce que l'on effectue la détermination à une température définie entre 15 et 35 °C.

**Claims**

1. Process for the determination of an opsonating, surface-binding $\alpha_2$-glycoprotein, wherein a buffered solution of antibodies is added against the opsonating surface-binding $\alpha_2$-glycoprotein using the sample to be tested, and the turbidity thus formed is determined at defined intervals of time either turbidimetrically or nephelometrically.

2. Process according to claim 1 wherein a buffer solution of pH 5 to 9.5 is used.

3. Process according to claim 2, wherein a buffer solution of pH 6.5 to 8.5 is used.

4. Process according to one of claims 1 to 3 above, wherein a water soluble polyethylene glycol is added.

5. Process according to claim 4, wherein 1 to 5% by weight of polyethylene glycol is added.

6. Process according to one of the claims above, wherein the determination is carried out at a certain temperature between 15 and 35 °C.

FIG.1

# FIG. 2

STANDARDKURVE von $\alpha_2$- SBOP am ABA 100

# FIG 3

KORRELATION $\dfrac{\text{ELEKTROIMMUNOASSAY ('ROCKET'-ELEKTROPHORESE)}}{\text{IMMUN-TURBIDIMETRISCHER TEST}}$

y = 1,179 x + 16,583

r = 0,844

9